(19) Europäisches Patentamt
European Patent Office
Office européen des brevets

(11) Veröffentlichungsnummer: **0 490 193 A1**

(12) **EUROPÄISCHE PATENTANMELDUNG**

(21) Anmeldenummer: **91120559.9**

(51) Int. Cl.5: **A61K 47/48**, A61K 31/19

(22) Anmeldetag: **29.11.91**

(30) Priorität: **30.11.90 DE 4038314**

(43) Veröffentlichungstag der Anmeldung:
**17.06.92 Patentblatt 92/25**

(84) Benannte Vertragsstaaten:
**AT BE CH DE DK ES FR GB GR IT LI LU NL SE**

(71) Anmelder: **MEDICE Chem.-Pharm. Fabrik**
**Pütter GmbH & Co. KG**
**Postfach 2063**
**W-5860 Iserlohn(DE)**

(72) Erfinder: **Szejtli, Jozsef, Prof.**
**c/o Fa. Cyclomed, Research & Dev.**
**Laboratories**
**Postfach 17, 1525 Budapest(HU)**
Erfinder: **Pütter, Sigurd, Dr.**
**c/o Fa. Medice, Pütter GmbH & Co. KG.,**
**Kuhloweg 37-38, 5860 Iserlohn(DE)**

(74) Vertreter: **Reinhard, Skuhra, Weise**
**Friedrichstrasse 31**
**W-8000 München 40(DE)**

(54) **Komplexe des aktiven Enantiomers des Ibuprofens mit Cyclodextrin.**

(57) Einschlußkomplex des aktiven S-(+)-Enantiomers des Ibuprofens und/oder seiner physiologisch verträglichen Salze mit einem Cyclodextrin und/oder einem Cyclodextrinderivat, ein Verfahren zu seiner Herstellung und die Verwendung als pharmazeutische Zubereitung.

EP 0 490 193 A1

Die Erfindung betrifft Komplexe des aktiven Enantiomers des Ibuprofens (S-(+)-Ibuprofen) mit Cyclodextrin, ein Verfahren zu ihrer Herstellung und ihre Verwendung als pharmazeutische Zubereitung.

S-(+)-Ibuprofen (S-(+)-2-(-4-Isobutylphenyl)propionsäure) ist ein gut bekanntes Medikament mit entzündungshemmenden, antipyretischen und analgetischen Eigenschaften. S-(+)-Ibuprofen ist eine kristalline Substanz von geringer Wasserlöslichkeit und Benetzbarkeit. Trotz dieser Tatsache wird Ibuprofen relativ schnell absorbiert und besitzt eine vollständige Bioverfügbarkeit nach oraler Einnahme (Albert, K.S., Gernaat, C.M. (1984): Anm. J. Med., 77, 40). Verschiedene Berichte zeigten Schwankungen in der Absorptionsgeschwindigkeit fester, oraler Darreichungsformen des Ibuprofens (Gillespie, W.R., DiSanto, A.R., Monovich, R.E., ALbert, K.S. (1982): *J. Pharm. Sci.,* 71, 1034; Stead, J.A., Freeman, M., John, E.G., Ward, G.T., Whiting, B. (1983): *Int. J. Pharm.,* 14, 59).

Das Ibuprofen-Razemat wird in verschiedenen, käuflich erwerbbaren Arzneimittelzubereitungen verwendet (z. B. Brufen (Klinge), Aktren (Bayer)), obwohl $\alpha$-Methylarylessigsäureanaloga in ihren pharmakodynamischen Eigenschaften eine deutlich Stereospezifität aufweisen, weil nämlich nur die S-(+)-Enantiomere die Prostaglandinsynthese hemmen. Weiterhin wurde die stereoselektive Disposition der Enantiomere in synovialer Flüssigkeit und im Plasma bei Patienten mit Arthritis untersucht. Der Gehalt des aktiven S-Enantiomers in der synovialen Flüssigkeit lag zu allen Zeiten bei allen Patienten über demjenigen des R-Enantiomers, wobei das Konzentrationsverhältnis S zu R 2,1 betrug (Day, R. Williams, K., Graham, G., Lee, E., Knihinicki, R., Champion, D. (1988): *Clin. Pharmacol. Ther. (St. Louis)* 43(5), 480). Diese Beobachtungen deuten auf die Möglichkeit einer wesentlichen Dosisverringerung des Ibuprofens, wodurch die Nebenwirkungen verringert würden.

Cyclodextrine sind zyklische Oligomere der Glucose mit apolaren Innenoberflächen, die in der Lage sind, hydrophobe Moleküle zu binden. Cyclodextrine sind kristallin, was ebenso für ihre Komplexe mit Fremdmolekülen gilt. Diese Komplexe zeigen etwas verbesserte Wasserlöslichkeitseigenschaften im Vergleich zu den Arzneimitteln selbst. Die letztgenannten Verbesserungen bilden den Gegenstand verschiedener Übersichtsartikel und von Patenten (Szejtli, J., *Cyclodextrin Technology,* Kluwer Academic Publishers, Dordrecht, Boston, London 1988; Pitha, J., Szente, L., Szejtli, J., *Molecular Encapsulation of Drugs by Cyclodextrins and Congeners,* in Controlled Drug Delivery, CRC Press Inc., (ed. Bruck, S.D.), Boca Raton, FL, 1983, pp. 125-148).

Die Komplexbildung des racemischen Ibuprofens und der Cyclodextrine wurde bereits detailliert in der Literatur untersucht. Ein fester racemischer Ibuprofen-$\beta$-Cyclodextrin-Komplex ist durch verschiedene Methoden herstellbar. Kurozumi et. al. (Kurozumi, M., Nambu, N., Nagai, T. (1975): Chem. Pharm. Bull. 23 (12) 3062) stellten eine Einschlußverbindung mit verschiedenen nicht-steroidalen, entzündungshemmenden Arzneimitteln einschließlich Ibuprofen mit $\alpha$- und $\beta$-Cyclodextrin aus einer homogenen Lösung durch Copräzipitations- und Gefriertrocknungsverfahren her. Es wurde gefunden, daß die Bioverfügbarkeit der hergestellten Erzeugnisse im Vergleich zu den Arzneimitteln selbst besser war. Nach Ibuprofengaben konnten aus dem Urin 9,7 % des Ibuprofen-$\beta$-Cyclodextrin-Komplexes und 5,0 % des freien Ibuprofens zurückgewonnen werden (Nambu, N., Shimoda, M., Takahashi, Y., Ueda, H., Nagai, T. (1978): *Chem. Pharm. Bull.* 26 (10) 2952).

Die Herstellung des (R,S)-Ibuprofen-$\beta$-Cyclodextrin-Komplexes in einem 1:1 Molverhältnis ist beschrieben in Japan Kokai JP 80,92,431 (Kakenyaku Kako Co., Ltd. 1980). Ibuprofen und $\beta$-Cyclodextrin wurden in einer 2%igen wäßrigen Natriumhydroxidlösung gelöst. Nach Neutralisierung der Lösung mit HCl wurde der Komplex präzipitiert. Dieser Ibuprofen-$\beta$-Cyclodextrin-Komplex zeigt geringere Störungen der Schleimhautmembranen und einen besseren Geruch als das reine Ibuprofen.

In Japan Kokai JP 81,46,837 (Kowa Pharm. Industry Co., Ltd., 1981) ist ein Sprühtrocknungsverfahren zur Beschichtung der Ibuprofenteilchen mit $\beta$-Cyclodextrin beschrieben, um den bitteren Geschmack zu verhindern. 2,0 g Ibuprofen, 5,7 g $\beta$-Cyclodextrin und 200 ml Wasser wurden bei 80° C gemischt und diese Mischung sprühgetrocknet. Das Molverhältnis dieses Produktes beträgt 2 Mole Ibuprofen auf 1 Mol $\beta$-Cyclodextrin.

Chow et. al. (Chow, D.D., Karara A.H. (1986): *Int. J. Pharm.* 28 95; Chow, D.D. (1986): *Diss. Abstr. Int.B 1987,* 47 (7) 2858) stellten einen festen Komplex durch das Lösen von $2x10^{-3}$ M (R,S)-Ibuprofen und $16x10^{-3}$ M $\beta$-Cyclodextrin in Wasser her. Der präzipitierte Komplex wurde gefiltert, gewaschen und bei 40° C über Nacht getrocknet. Das stöchiometrische Verhältnis des festen Komplexes betrug 2:3 (Ibuprofen zu $\beta$-Cyclodextrin), was auf die Beiträge verschiedener Komplexe, die im System präzipitieren, hinweist. Bioverfügbarkeitsuntersuchungen bei Ratten zeigten, daß der Absorptionsgrad sowohl für das freie als auch für das komplexierte Ibuprofen gleich war. Das komplexierte Ibuprofen erreichte jedoch die Spitzen-Plasmakonzentration um das 2,5-fache schneller als das Arzneimittel allein.

Cyclodextrin-(R,S)-Ibuprofen-Komplexe wurden weiterhin durch Markarian et.al. (Markarian, B.M., Choen, G.L.(1988): Eur.Pat. Appl. EP 274 444) durch Mischen von (R,S)-Ibuprofen und einem Cyclodextrin in

einer Lösung bei erhöhten Temperaturen hergestellt. Die Löslichkeit des Ibuprofens war wesentlich erhöht.

Auch in weiteren Veröffentlichungen ist der Einfluß von Cyclodextrinen oder Cyclodexgrinderivaten auf die Löslichkeits- und Diffusionsgeschwindigkeit des Ibuprofens beschrieben (Nenard, F.A., Dedhiya, M.G., Rhodes, C.T. (1988): Drug. Dev. Ind. Pharm. 14 (11) 1529; Szemán, J. Szente, L., Szabó, T., Szejtli, J.: Proceedings of the Fourth Int. Symp. on Cyclodextrins, Kluwer Academic Publishers, (Eds.: Huber, O., Szejtli, J.), Dordrecht, 1988 p. 393; Orienti, I., Cavallari, C., Zecchi, V. (1989):Arch. Pharm. (Weinheim) 322 207).

Aus der EP 0 346 006 sind pharmazeutische Zubereitungen bekannt, die Komplexe des $\beta$-Cyclodextrins mit Ibuprofensalzen enthalten. Die Verwendung von S-(+)-Ibuprofen in freier Form als auch die Verwendung von S-(+)-Ibuprofen-Salzen in Verbindung mit Cyclodextrinen wird in dieser Anmeldung nicht beschrieben oder nahegelegt. Die aus dem Stand der Technik bekannten Cyclodextrin-Ibuprofen-Komplexe verwenden ausnahmslos die razemischen Gemische des Ibuprofens und zeigen den Nachteil, daß hohe Dosen an (R,S)-Ibuprofen mit den damit verbundenen Nachteilen eingesetzt werden müssen.

Es ist eine Aufgabe der Erfindung, neue Komplexe des S-(+)-Ibuprofens bereitzustellen.

Diese Aufgabe wird erfindungsgemäß dadurch gelöst, daß ein Einschlußkomplex des aktiven S-(+)-Enantiomers des Ibuprofens und/oder seiner physiologisch verträglichen Salze mit einem Cyclodextrin und/oder einem Cyclodextrinderivat bereitgestellt wird. Weiterhin wird ein Verfahren zur Herstellung dieser Cyclodextrin-Komplexe offenbart.

Die erfindungsgemäß bereitgestellten Komplexe aus S-(+)-Ibuprofen und Cyclodextrin weisen eine gute Bioverfügbarkeit und einen besseren Geschmack auf, als die Komplexe des Standes der Technik.

Erfindungsgemäß ist das Cyclodextrin eine Verbindung der folgenden Formel (I)

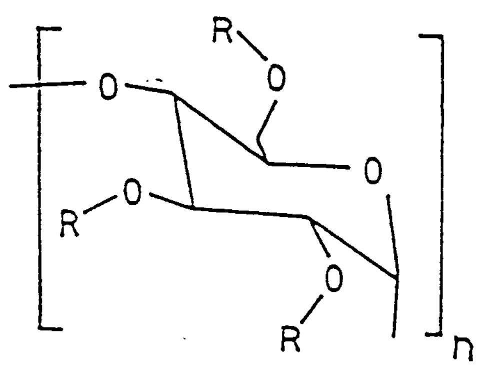

wobei

n = 6,7 oder 8

R = H und/oder ein Alkylrest ($C_1$ - $C_5$), Aminoalkyl ($C_1$ - $C_5$), Alkyl- oder Dialkyl($C_1$ - $C_5$)-aminoalkyl ($C_1$ - $C_5$), mit Ausnahme des Diethylaminoethylsubstituenten, oder ein Cyclodextrin-Ether-Derivat.

Das Cyclodextrinderivat kann ein gemischter Ether sein. Die durchschnittliche Zahl an Substituenten am Cyclodextrinring (PS) kann im Bereich von 0-21 liegen. Das Verhältnis des S-(+)-Ibuprofens zum Cyclodextrin liegt im Bereich von 0,01 bis 2,00 Gewichtsteilen.

Die $C_1$ - $C_5$-Alkylgruppe der Formel (I) ist unverzweigt oder verzweigt und ist bevorzugt eine $C_1$ - $C_3$-Alkylgruppe, insbesondere bevorzugt eine Methyl-, Ethyl-, Propyl- oder Isopropylgruppe. Die Aminoalkyl- und die Alkyl- oder Dialkylaminoalkylgruppen können eine unverzweigte oder verzweigte $C_1$ - $C_5$-Alkylgruppe enthalten, bevorzugt eine $C_1$ - $C_3$-Alkylgruppe, insbesondere bevorzugt eine Methyl-, Ethyl-, Propyl- oder Isopropylgruppe. Die Diethylaminoehtylgruppe wird ausschließlich in gemischten Cyclodextrin-Ether-Derivaten verwendet.

Der Substitutionsgrad PS der Cyclodextrinderivate kann im Bereich von 0 - 21, in den gemischten Ethern kann der PS des Alkylsubstituenten bevorzugt 0 - 14 betragen und die Summe der Substituenten liegt bevorzugt bei 0 - 21.

Die Cyclodextrinverbindung kann weiterhin ein dihydroxylalkyliertes Polymerderivat, ein wasserlösliches Cyclodextrinpolymer (Szejtli, J., Zsadon, B., Fenyvesi, E., Szilasi, M., Tüdos, F. (1982) : Hung. Teljes HU 180,597) oder ein ionisches, wasserlösliches Cyclodextrinpolymer (Szejtli, J., Fenyvesi, E., Zsadon, B., Szilasi, M., Décsei L. (1984): Eur. Pat. Appl. EP 119 453) sein. Das ionische Polymer ist bevorzugt ein Aminoalkyl-($C_1$ - $C_3$)- oder Dialkyl($C_1$ - $C_3$)-aminoalkyl($C_1$ - $C_3$)-substituiertes Polymer.

Das Gewichtsverhältnis des S-(+)-Ibuprofens und des Cyclodextrins liegt im Bereich von 0,01 bis 2,0,

weiterhin bevorzugt zwischen 0,01 und 0,25, zwischen 0,5 bis 2,0 und zwischen 0,1 bis 0,8; insbesondere bevorzugt beträgt das Verhältnis von Cyclodextrin zu S-(+)-Ibuprofen 1:1.

Eine weitere Aufgabe der Erfindung ist die Schaffung eines Verfahrens zur Herstellung von Ibuprofen.

Erfindungsgemäß wird der S-(+)-Ibuprofen-Cyclodextrin-Komplex durch eine Phasentransformation hergestellt, wobei das S-(+)-Ibuprofen in einer wäßrigen Suspension geeigneter Cyclodextrine als Wirtsmoleküle geschmolzen wird.

Das Fremdmolekül wird in einer wäßrigen Suspension des Cyclodextrins über seinem Schmelzpunkt gerührt, wodurch ein kristalliner Einschlußkomplex entsteht. Unter kräftigem Rühren wird das geschmolzene Fremdmolekül in Form kleiner Tröpfchen in der Cyclodextrinsuspension dispergiert, wobei ein Emulsions-Suspensions-Systementsteht. Nach 20 - 30 Stunden ist die Komplexierung vollständig, und zwar abhängig von der Rührintensität, dem Probenmaßstab, der Temperatur usw.. Wenn das Reaktionssystem zu einer einfachen Suspension wird, ist die Komplexierung vollständig. Anschließend wird die Suspension abgekühlt, um die Löslichkeit des Komplexes zu vermindern. Nach der Filtrierung wird der kristalline Komplex getrocknet.

Unter physiologischen Bedingungen wurde gefunden, daß die Wasserlöslichkeit des nach obigem Verfahren hergestellten S-(+)-Ibuprofen-$\beta$-Cyclodextrin-Komplexes das neunfache des nichtkomplexierten Ibuprofens übersteigt. Weiterhin wird in dieser Zubereitung der unerwünschte Geruch, Geschmack und die störende Wirkung auf die Schleimhautmembran des Ibuprofens vermindert. Die gute Wasserlöslichkeit, die verminderte Kristallinität und die verbesserte Benetzbarkeit des derart hergestellten S-(+)-Ibuprofen-Cyclodextrin-Komplexes resultiert in besseren Löslichkeitseigenschaften (Tabelle I) und ergab die Möglichkeit der Anwendung der Verbindung in neuen pharmazeutischen Zubereitungen des S-(+)-Ibuprofen, z.B. in Form von Brausetabletten, Sirupen oder Suspensionen.

S-(+)-Ibuprofen ist ein ideales Fremdmolekül für diese Komplexierungsverfahren, da es einen niedrigen Schmelzpunkt von 50° - 53°C besitzt. Für die Komplexierung können verschiedene Arten an Cyclodextrinen oder Cyclodextrinderivaten verwendet werden.

Das Verfahren besitzt im Vergleich zu den aus dem Stand der Technik bekannten folgende Vorteile :

- Es werden keine organischen Lösungsmittel verwendet. Normalerweise werden in der Reaktionsmischung organische Lösungsmittel verwendet, wenn der Komplex in einer homogenen Lösung oder Suspension hergestellt wird.
- Das Volumen der Reaktionsmischung ist relativ gering, weil das Cyclodextrin nicht gelöst vorliegt.
- Das erfindungsgemäße Verfahren ist sehr einfach (Rühren, Filtrieren, Trocknen).
- Dieses Verfahren kann leicht in großem Maßstab unter industriellen Bedingungen durchgeführt werden.
- Der Wirkstoffverlust ist nur gering und es findet keine Verschmutzung der Umwelt statt.
- Erfindungsgemäß wird kein Überschuß des Fremdmoleküls benötigt, wie dies beim Komplexierungsverfahren durch Schmelzen notwendig ist.

Um die gute biologische Verfügbarkeit des S-(+)-Ibuprofens aus der komplexierten Form zu testen, wurden in vitro-Diffusionsversuche durchgeführt. Die Diffusion des S-(+)-Ibuprofens aus dem $\beta$-Cyclodextrin-Komplex war schneller, die S-(+)-Ibuprofen-Menge in der Rezeptorzelle betrug das 1,6fache bei pH = 1,2 nach 6 Stunden im Vergleich zum freien Arzneimittel (Tabelle II).

Weitere bevorzugte Ausführungsformen der Erfindung ergeben sich auch aus den anliegenden Patentansprüchen.

## Beispiele

### Beispiel 1

Herstellung von S-(+)-Ibuprofen mit einer verbesserten Wasserlöslichkeit unter Verwendung von $\beta$-Cyclodextrin zum molekularen Einschluß

Der S-(+)-Ibuprofen-$\beta$-Cyclodextrin-Komplex wird in einem molaren Verhältnis von 2 bis 3,5 hergestellt, wobei der 20%ige Überschuß des $\beta$-Cyclodextrins in der Reaktionsmischung die Verschiebung des Gleichgewichts zur Einschlußkomplexierung erleichtert.

Ein 2000-ml-Dreihalskolben wird mit 1100 ml Wasser gefüllt und auf 60° C im Wasserbad erwärmt. Unter kräftigem Rühren werden 55 g (0,27 mol) S-(+)-Ibuprofen zum Wasser zugegeben. Nachdem das geschmolzene S-(+)-Ibuprofen in einer Emulsion vorliegt, werden 626 g $\beta$-Cyclodextrin (0,475 mol, Feuchtigkeitsgehalt 13,8%) zugegeben. Die erhaltene Emulsions-Suspension wird bei 60° C und 600 rpm gerührt. Nach diesem Einschlußverfahren wird das eingeschlossene S-(+)-Ibuprofen analysiert. Die Komplexierung ist innerhalb von etwa 24 Stunden vollständig. Danach wird die Suspension langsam auf 5° C abgekühlt. Das Produkt wird auf einem Glasfilter unter Vakuum filtriert. Der kristalline Komplex wird bei 60° C zwei Stunden lang getrocknet.

Der S-(+)-Ibuprofengehalt des derart hergestellten Produktes beträgt 9,7 ± 0,25 %, der Gewichtsverlust beträgt ca. 5 - 6 %. Die Menge an freiem S-(+)-Ibuprofen im hergestellten Komplex beträgt etwa 0,6 - 0,8 g/100g Produkt, bestimmt durch die Wärmefreisetzungsanalyse.Der Abbau und die Verdampfung des S-(+)-Ibuprofen werden im Temperaturbereich von 100° C - 165° C auf den TEA-Kurven des freien S-(+)-Ibuprofens und seiner Mischung mit β-Cyclodextrin dargestellt (Figur 1). Die meßbare Menge einer flüchtigen organischen Verbindung aus dem S-(+)-Ibuprofen-β-Cyclodextrin-Komplex beträgt nur 0,6 - 0,8 mg/g in einem Temperaturbereich von 100° C bis 180° C, wodurch die Komplexbildung des S-(+)-Ibuprofens bestätigt wird. Der Komplex zeigt eine gute Löslichkeit und gute Auflösungseigenschaften (vgl. Tabelle I).

Beispiel 2

Herstellung von molekular eingeschlossenem S-(+)-Ibuprofen-β-Cyclodextrin

55 g (0,27 mol) S-(+)-Ibuprofen und 626 g (0,75 mol) β-Cyclodextrinwerden zu 500 ml Wasser gegeben. Das Reaktionsgemisch wird bei 60° C mit einem Ultra-Turrax (10 000 rpm) eine Stunde lang gerührt. Anschließend wird die Suspension auf 5° C abgekühlt. Das feste Produkt wird gefiltert und bei 60° C getrocknet.

Beispiel 3

Herstellung von S-(+)-Ibuprofen mit verbesserter Wasserlöslichkeit unter Verwendung von gamma-Cyclodextrin zum molekularen Einschluß

Ein 1000 ml Dreihalskolben wird mit 400 ml Wasser gefüllt und im Wasserbad auf 60° C erwärmt. Unter kräftigem Rühren werden 55 g (0,27 mol) S-(+)-Ibuprofen zum Wasser gegeben.

Nach Emulgierung des geschmolzenen Ibuprofens werden 616 g (0,475 mol) gamma-Cyclodextrin zugegeben. Die erhaltene Emulsionssuspension wird mit 600 rpm bei 60° C gerührt. Auf das Einschlußverfahren folgt die Analyse des eingeschlossenen Ibuprofens. Die Komplexierung ist nach etwa 24 Stunden beendet. Die Suspension wird anschließend langsam auf 5° C abgekühlt. Das Produkt wird auf einem Glasfilter in vacuo filtriert. Der kristalline Komplex wird bei 60° C zwei Stunden lang getrocknet. Die Wasserlöslichkeit des S-(+)-Ibuprofens beträgt 0,55 mg/ml für dieses Produkt.

Beispiel 4

Herstellung von molekular eingeschlossenem S-(+)-Ibuprofen unter Verwendung von 2,6-Di-0-methyl-β-cyclodextrin

450 ml Wasser werden auf 60° C erwärmt. 20,6 g (0,1 mol) S-(+)-Ibuprofen und 133,3 g (0,1 mol) 2,6-Di-0-methyl-β-cyclodextrin (DIMEB) werden unter kräftigem Rühren zugegeben. Die erhaltene Emulsions-Suspensions-Mischung wird mit 600 rpm bei 60° C gerührt. Das Komplexierungsverfahren ist innerhalb von etwa 24 Stunden beendet. Anschließend wird die warme Suspension auf einem warmen Glasfilter in vacuo gefiltert. Das Produkt wird bei 60° C zwei Stunden lang getrocknet. Der S-(+)-Ibuprofengehalt des Produktes beträgt 16,6 %. Die Wasserlöslichkeit des S-(+)-Ibuprofen wird auf 21 mg/ml erhöht, was eine etwa 200fache Zunahme der Löslichkeit bedeutet.

Beispiel 5

Herstellung von molekular eingeschlossenem S-(+)-Ibuprofen unter Verwendung eines polymeren hydroxyalkylierten Cyclodextrinderivates

20,6 g S-(+)-Iboprofen und 300 g wasserlösliches Cyclodextrinpolymer (CDPS) werden in 400 ml Wasser bei 60° C gemischt. Die Reaktionsmischung wird 10 Stunden lang gerührt und anschließend sprühgetrocknet. Der S-(+)-Ibuprofengehalt des Produktes beträgt 6,8 %. Die Wasserlöslichkeit des S-(+)-Ibuprofens beträgt 8,0 mg/ml.

Beispiel 6

Herstellung von S-(+)-Ibuprofen mit verbesserter Wasserlöslichkeit unter Verwendung eines ionischen Cyclodextrinpolymeren

20,6 g S-(+)-Ibuprofen und 300 g eines durch Diethylaminogruppen substituierten Cyclodextrinpolymeren werde in 400 ml Wasser bei 60° C vermischt. Die Reaktionsmischung wird zwei Stunden lang gerührt und anschließend sprühgetrocknet. Der S-(+)-Ibuprofengehalt des Produktes beträgt 6,8 %.

Beispiel 7

Herstellung von S-(+)-Ibuprofen-Brausetabletten

| 1 Tablette enthält in mg: | |
|---|---|
| S-(+)-Ibuprofen-Komplex* aus Beispiel 1 (entspricht 200 mg S-(+)-Ibuprofen) | 2065,0 |
| Lactose | 700,0 |
| Citronensäure | 150,0 |
| Natriumdihydrogencitrat | 390,0 |
| Natriumcarbonat | 125,0 |
| Natriumhydrogencarbonat | 500,0 |
| Magnesiumstearat | 40,0 |
| Zitronenaroma (Fa. Dragoco, Nr.9/021907) | 0,8 |
| Natriumcyclamat | 20,0 |

* Der Wirkstoffgehalt kann variiert werden von 100 - 200 mg S-(+)-Ibuprofen pro Tablette. Die Hilfsstoffe werden entsprechend reduziert bzw. erhöht.

Beispiel 8

Herstellung von S-(+)-Ibuprofen-Brausetabletten

| 1 Tablette enthält in mg: | |
|---|---|
| S-(+)-Ibuprofen-Komplex* aus Beispiel 4 (entspricht 200 mg S-(+)-Ibuprofen) | 1205,0 |
| Lactose | 400,0 |
| Citronensäure | 150,0 |
| Weinsäure | 300,0 |
| Natriumhydrogencarbonat | 375,0 |
| Magnesiumstearat | 25,0 |
| Zitronenaroma (Fa. Dragoco, Nr.9/021907) | 0,5 |

* Der Wirkstoffgehalt kann variiert werden von 100 - 200 mg S-(+)-Ibuprofen pro Tablette. Die Hilfsstoffe werden entsprechend reduziert bzw. erhöht.

Beispiel 9

Herstellung von S-(+)-Ibuprofen-Suspension

| 100 g Suspension enthalten : | |
|---|---|
| S-(+)-Ibuprofen-Komplex* aus Beispiel 1 (entspricht 2000 mg S-(+)-Ibuprofen) | 20,65 g |
| Sorbit 70 % - Lösung | 10,00 g |
| Natriumcyclamat | 0,40 g |
| Krauseminz-Aroma (Haarmann & Reimer 290057/89658) | 0,10 g |
| Methylcellulose MHB 1000 | 2,00 g |
| Sorbinsäure | 0,15 g |
| Gereinigtes Wasser | 66,70 g |

Beispiel 10

Herstellung von S-(+)-Ibuprofen-Sirup

| 100 g Sirup enthalten : | |
|---|---|
| S-(+)-Ibuprofen-Komplex* aus Beispiel 4 (entspricht 2,00 g S-(+)-Ibuprofen) | 12,05 g |
| Sorbit 70 % - Lösung | 14,30 g |
| Kirscharoma (Haarmann & Reimer 203816) | 0,02 g |
| Sorbinsäure | 0,15 g |
| Gereinigtes Wasser | 73,48 g |

Tabelle I. Auflösungseigenschaften des S-(+)-Ibuprofens und des S-(+)-Ibuprofen-ß-Cyclodextrin-Komplexes in Wasser bei 37° C

| Zeit (min) | 1 | 5 | 10 | 15 | 30 | 60 |
|---|---|---|---|---|---|---|
| gelöstes Ibuprofen aus der reinen Substanz (mg/ml) | 0,04 | 0,10 | 0,12 | 0,13 | 0,12 | 0,12 |
| gelöstes Ibuprofen aus dem S-(+)-Ibuprofen-ß-Cyclodextrin-Komplex (mg/ml) | 0,86 | 0,78 | 0,81 | 0,79 | 0,80 | 0,81 |

Tabelle II. Diffusionsstudie

Konzentration von S-(+)-Ibuprofen in der Rezeptorzelle in Abwesenheit und in Gegenwart von ß-Cyclodextrin in einer Pufferlösung (pH = 1,2) bei 37° C

| Zeit (min) | 90 | 120 | 180 | 240 | 360 |
|---|---|---|---|---|---|
| S-(+)-Ibuprofen (mg/ml) | 0,022 | 0,027 | 0,038 | 0,047 | 0,061 |
| S-(+)-Ibuprofen-ß-Cyclodextrin-Komplex (mg/ml) | 0,030 | 0,038 | 0,054 | 0,068 | 0,101 |

## Patentansprüche

1. Einschlußkomplex des aktiven S-(+)-Enantiomers des Ibuprofens und/oder seiner physiologisch verträglichen Salze mit einem Cyclodextrin und/oder einem Cyclodextrinderivat.

**2.** Einschlußkomplex nach Anspruch 1, dadurch gekennzeichnet, daß das Ibuprofen ein freies S-(+)-Ibuprofen ist.

**3.** Einschlußkomplex nach Anspruch 1 oder 2, dadurch gekennzeichnet, daß das Cyclodextrin die folgende Formel aufweist :

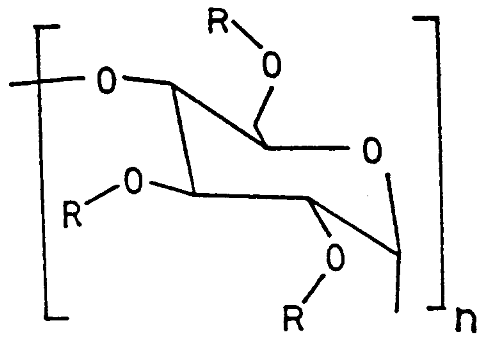

wobei n = 6 (α-Cyclodextrin), 7 (β-Cyclodextrin) oder 8 (γ-Cyclodextrin) R = H und/oder eine Alkyl- und/oder eine Aminoalkyl- und/oder eine Alkylaminoalkylgruppe oder Dialkyl-aminoalkylgruppe ist.

**4.** Einschlußkomplex nach einem oder mehreren der vorhergehenden Ansprüche, dadurch gekennzeichnet, daß R = H und/oder eine $C_1$ - $C_5$-Alkylgruppe und/oder eine $C_1$ - $C_5$ Aminoalkylgruppe und/oder eine $C_1$ - $C_5$-Alkyl- oder Dialkyl-$C_1$-$C_5$-Aminoalkylgruppe ist.

**5.** Einschlußkomplex nach einem oder mehreren der vorhergehenden Ansprüche, dadurch gekennzeichnet, daß das Cyclodextrinderivat ein Misch-Cyclodextrin-Etherderivat ist.

**6.** Einschlußkomplex nach einem oder mehreren der vorhergehenden Ansprüche, dadurch gekennzeichnet, daß die Diethylaminoethylgruppe ausschließlich in Misch-Cyclodextrin-etherderivaten vorliegt.

**7.** Einschlußkomplex nach einem oder mehreren der vorhergehenden Ansprüche, dadurch gekennzeichnet, daß R eine unverzweigte oder verzweigte $C_1$ - $C_5$-Alkylgruppe, bevorzugt eine $C_1$ - $C_3$-Alkylgruppe, ist.

**8.** Einschlußkomplex nach einem oder mehreren der vorhergehenden Ansprüche, dadurch gekennzeichnet, daß R eine Methyl-, Ethyl-, Propyl- oder Isopropylgruppe ist.

**9.** Einschlußkomplex nach einem oder mehreren der vorhergehenden Ansprüche, dadurch gekennzeichnet, daß R eine Aminoalkyl-, eine Alkyl- oder Dialkylaminoalkylgruppe mit verzweigten oder unverzweigten $C_1$ - $C_5$-Alkylgruppen, bevorzugt eine $C_1$ - $C_3$-Alkylgruppe, ist.

**10.** Einschlußkomplex nach einem oder mehreren der vorhergehenden Ansprüche, dadurch gekennzeichnet, daß die Alkylgruppe eine Methyl, Ethyl-, Propyl- oder Isopropylgruppe ist.

**11.** Einschlußkomplex nach einem oder mehreren der vorhergehenden Ansprüche, dadurch gekennzeichnet,

daß das Cyclodextrin ein Dimethyl- oder Hydroxypropyl-$\beta$-Cyclodextrinist.

**12.** Einschlußkomplex nach einem oder mehreren der vorhergehenden Ansprüche, dadurch gekennzeichnet, daß das Cyclodextrin ein wasserlösliches, polymeres Cyclodextrin ist.

**13.** Einschlußkomplex nach einem oder mehreren der vorhergehenden Ansprüche, dadurch gekennzeichnet, daß das polymere Cyclodextrin 2 - 5 Cyclodextrineinheiten enthält.

**14.** Einschlußkomplex nach einem oder mehreren der vorhergehenden Ansprüche, dadurch gekennzeichnet, daß das Cyclodextrin ein polymeres, dihydroxyalkyliertes Cyclodextrinderivat ist.

**15.** Einschlußkomplex nach einem oder mehreren der vorhergehenden Ansprüche, dadurch gekennzeichnet, daß das dihydroxyalkylierte Cyclodextrinderivat durch ionische Gruppen substituiert ist.

**16.** Einschlußkomplex nach einem oder mehreren der vorhergehenden Ansprüche, dadurch gekennzeichnet, daß der Substitutionsgrad am Cyclodextrinring im Bereich von 0 - 21 liegt.

**17.** Einschlußkomplex nach einem oder mehreren der vorhergehenden Ansprüche, dadurch gekennzeichnet, daß der Substitutionsgrad bei Dimethyl-Cyclodextrin 14 ist.

**18.** Einschlußkomplex nach einem oder mehreren der vorhergehenden Ansprüche, dadurch gekennzeichnet, daß der Substitutionsgrad bei Hydroxypropylcyclodextrin im Bereich von 0,2 - 1 liegt.

**19.** Einschlußkomplex nach einem oder mehreren der vorhergehenden Ansprüche, dadurch gekennzeichnet, daß in Mischethern der Substitutionsgrad der Alkylsubstituenten bevorzugt im Bereich von 0 - 14 liegt, wobei die Summe der Substituenten im Bereich von 0 - 21 liegen kann.

**20.** Einschlußkomplex nach einem oder mehreren der vorhergehenden Ansprüche, dadurch gekennzeichnet, daß das Gewichtsverhältnis des S-(+)-Ibuprofens zum Cyclodextrin im Bereich von 0,01 - 2,0 liegt.

**21.** Einschlußkomplex nach einem oder mehreren der vorhergehenden Ansprüche, dadurch gekennzeichnet, daß das Gewichtsverhältnis des S-(+)-Ibuprofens zum Cyclodextrin im Bereich von 0,01 - 0,25 liegt.

**22.** Einschlußkomplex nach einem oder mehreren der vorhergehenden Ansprüche, dadurch gekennzeichnet, daß das Gewichtsverhältnis des S-(+)-Ibuprofens zum Cyclodextrin im Bereich von 0,5 - 2,0 liegt.

**23.** Einschlußkomplex nach einem oder mehreren der vorhergehenden Ansprüche, dadurch gekennzeichnet, daß das Gewichtsverhältnis des S-(+)-Ibuprofens zum Cyclodextrin im Bereich von 0,1 - 0,8 liegt.

**24.** Einschlußkomplex nach einem oder mehreren der vorhergehenden Ansprüche, dadurch gekennzeichnet, daß das Gewichtsverhältnis des S-(+)-Ibuprofens zum Cyclodextrin 1 : 1 beträgt.

**25.** Verfahren zur Herstellung des S-(+)-Ibuprofen-Cyclodextrin-Komplexes und/oder seiner physiologisch verträglichen Salze nach einem oder mehreren der Ansprüche 1 - 24, dadurch gekennzeichnet, daß das S-(+)-Ibuprofen in einer wäßrigen Lösung eines geeigneten Cyclodextrins oder Cyclodextrinderivates über seinem Schmelzpunkt gerührt wird, bis sich der kristalline Einschlußkomplex bildet.

**26.** Verfahren nach Anspruch 25,
dadurch gekennzeichnet,
daß der Schmelzpunkt im Bereich zwischen 45 °C - 200 °C liegt.

**27.** Verfahren nach einem oder mehreren der vorhergehenden Ansprüche,
dadurch gekennzeichnet,
daß der Schmelzpunkt im Bereich zwischen 45 °C - 120 °C liegt.

**28.** Verfahren nach einem oder mehreren der vorhergehenden Ansprüche,
dadurch gekennzeichnet,
daß der Schmelzpunkt des freien S-(+)-Ibuprofens bei 50 °C - 53 °C liegt.

**29.** Verfahren nach einem oder mehreren der vorhergehenden Ansprüche,
dadurch gekennzeichnet,
daß die Komplexbildung dann vollständig ist, wenn das Reaktionssystem als Suspension vorliegt.

**30.** Verfahren nach einem oder mehreren der vorhergehenden Ansprüche,
dadurch gekennzeichnet,
daß die Komplexbildung nach einem Zeitraum von 20 - 30 Stunden vollständig ist.

**31.** Verfahren nach einem oder mehreren der vorhergehenden Ansprüche,
dadurch gekennzeichnet,
daß die Suspension nach der Komplexierung abgekühlt wird, um die Löslichkeit des Komplexes zu vermindern.

**32.** Verfahren nach einem oder mehreren der vorhergehenden Ansprüche,
dadurch gekennzeichnet,
daß der kristalline Komplex filtriert und getrocknet wird.

**33.** Verwendung des Einschlußkomplexes nach einem oder mehreren der vorhergehenden Ansprüche als pharmazeutische Zubereitung.

**34.** Verwendung des Einschlußkomplexes nach einem oder mehreren der vorhergehenden Ansprüche als pharmazeutische Zubereitung in Form von Brausetabletten , einer Suspension oder eines Sirups.

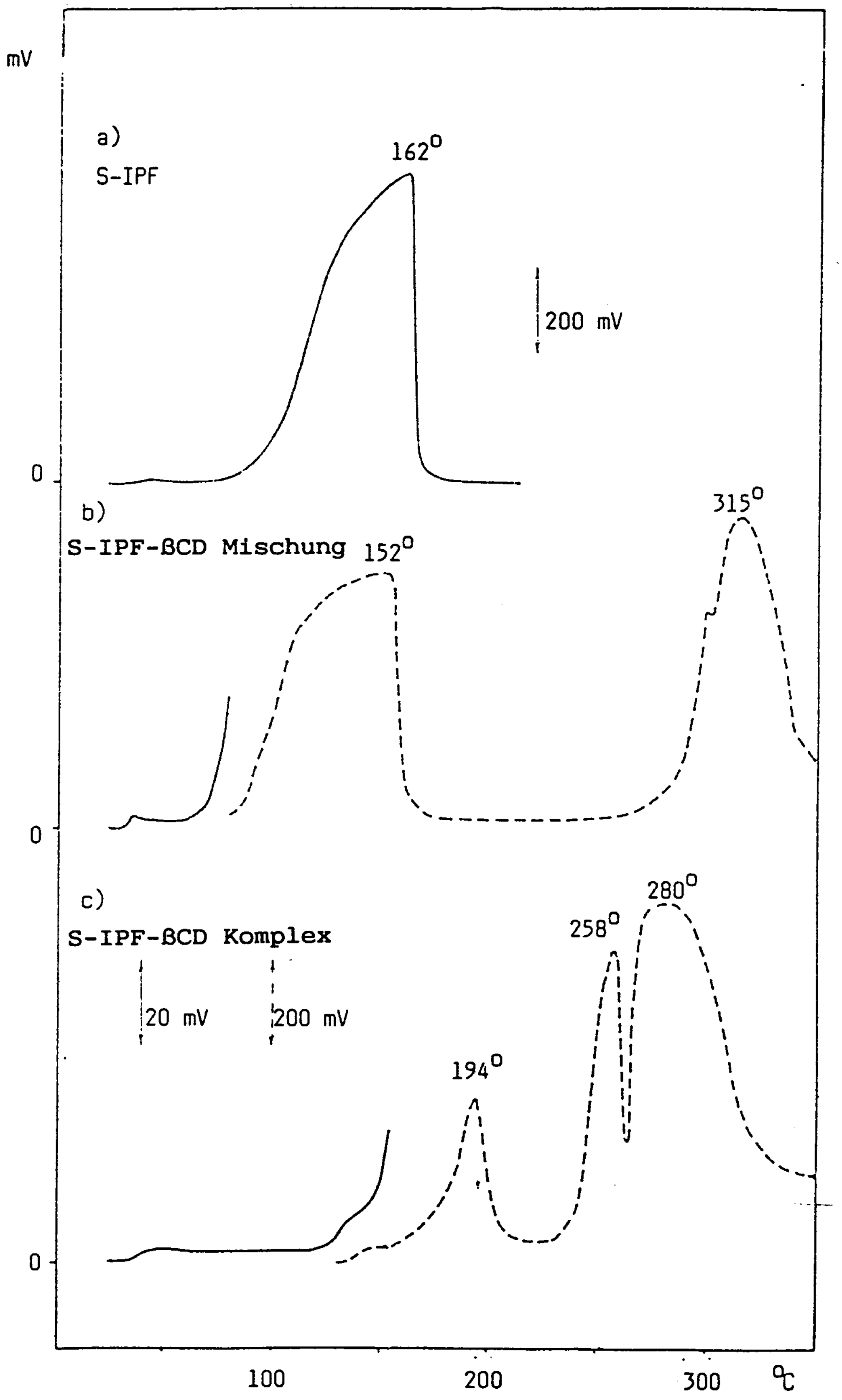


Figur 1. TEA-Kurven der S-(+)-Ibuprofen-ß-Cyclodextrin-Systeme

Europäisches Patentamt

# EUROPÄISCHER RECHERCHENBERICHT

Nummer der Anmeldung

EP 91 12 0559

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | DE-A-3 639 038 (MEDICE CHEM.-PHARM.)<br>* Ansprüche; Seite 3, Zeilen 59-66 * | 1-34 | A 61 K 47/48<br>A 61 K 31/19 |
| P,Y | EP-A-0 406 811 (AJINOMOTO CO.)<br>* Seite 3, Zeilen 13-16,20-26; Ansprüche * | 1-34 | |
| Y | WO-A-9 002 141 (AUSTRALIA COMM. RESEARCH)<br>* Seite 23, Zeile 12; Seite 30, Absatz 4; Seite 31, Absatz 1; Seite 49, Absatz 1; Seite 75, Beispiele 25,26; Ansprüche 57,106 * | 1-34 | |
| D,A | EP-A-0 274 444 (BRISTOL-MYERS CO.)<br>* Seite 3, Zeilen 12-19; Ansprüche * | 1-34 | |
| A | STN FILE SERVER, KARLSRUHE, DE, CHEMICAL ABSTRACTS, Band 114, Nr. 6, Zusammenfassung Nr. 49684n, Columbus, Ohio, US; A. BERTHOD et al.: "Cyclodextrin chiral stationary phases for liquid chromatographic separations of drug stereoisomers", & J. PHARM. BIOMED. ANAL., 8(2), 123-30<br>* Ganze Zusammenfassung * | 1-34 | RECHERCHIERTE SACHGEBIETE (Int. Cl.5)<br>A 61 K |

Der vorliegende Recherchenbericht wurde für alle Patentansprüche erstellt

| Recherchenort | Abschlußdatum der Recherche | Prüfer |
|---|---|---|
| DEN HAAG | 17-03-1992 | BERTE M.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)